# EUROPÄISCHE PATENTSCHRIFT

(11) **EP 3 662 261 B1**
(45) Veröffentlichungstag und Bekanntmachung des Hinweises auf die Patenterteilung: **02.06.2021**
(21) Anmeldenummer: 18746640.4
(22) Anmeldetag: 18.07.2018
(51) Int. Cl.: G01N 21/33, F01N 11/00, G01N 21/3504, G01N 33/00

(54) **GASANALYSATOR ZUR MESSUNG VON STICKOXIDEN UND SCHWEFELDIOXID IN ABGASEN**
GAS ANALYZER FOR MEASURING NITROGEN OXIDES AND SULFUR DIOXIDE IN EXHAUST GASES
ANALYSEUR DE GAZ POUR MESURER LES OXYDES D'AZOTE ET LE DIOXYDE DE SOUFRE DANS DES GAZ D'ÉCHAPPEMENT

(30) Priorität: 31.07.2017 DE 102017213196
(43) Veröffentlichungstag der Anmeldung: 10.06.2020
(73) Patentinhaber: Siemens Aktiengesellschaft, 80333 München (DE)
(72) Erfinder: HEFFELS, Camiel, 76297 Stutensee-Büchig (DE); LANG, Jochen, 76185 Karlsruhe (DE); SCHMIDT, Benjamin, 76187 Karlsruhe (DE)
(86) Internationale Anmeldenummer: PCT/EP2018/069486
(87) Internationale Veröffentlichungsnummer: WO 2019/025186

(56) Entgegenhaltungen:
- WO-A1-2015/181879
- CN-A- 105 424 631
- DE-U1-202017 001 743
- JP-A- S60 119 443
- JP-A- 2012 026 331
- US-A1- 2013 045 541
- US-A1- 2016 010 493

## Beschreibung

Die Erfindung betrifft einen Gasanalysator zur Messung von Stickoxiden und Schwefeldioxid in einem Abgas

Aus Ryoichi Higashi et al.: "A NOx and SO2 gas analyzer using deep-UV and violet light-emitting diodes for continuous emissions monitoring systems", Proc. SPIE 9003, Light-Emitting Diodes: Materials, Devices, and Applications for Solid State Lighting XVIII, 90031F (February 27, 2014), ist ein Gasanalysator zur Messung von Stickoxiden und Schwefeldioxid in einem Abgas bekannt. Vor der Analyse wird das Abgas in zwei Stufen behandelt, wobei in einer ersten Stufe in dem Abgas enthaltenes Stickstoffmonoxid mit Hilfe von Ozon in mit dem Gasanalysator messbares Stickstoffdioxid umgesetzt wird. Das Ozon wird mittels elektrischer Entladung aus Luftsauerstoff erzeugt und dem Abgas zugeführt. In einer zweiten Behandlungsstufe wird das Abgas auf etwa 300 °C erhitzt, um überschüssiges Ozon und durch Reaktion von Stickstoffdioxid und Ozon entstandenes und von dem Gasanalysator nicht messbares Distickstoffpentoxid (N2O5) thermisch in Stickstoffdioxid zu zersetzen. Die von dem Gasanalysator ermittelte Konzentration von Stickstoffdioxid ist somit ein Maß für die Konzentration von Stickoxiden in dem Abgas.

Bei dem bekannten Gasanalysator sind eine MUV-Leuchtdiode mit einer Emissionswellenlänge von 280 nm und eine NUV-Leuchtdiode mit einer Emissionswellenläge von 400 nm dicht nebeneinander in einem LED-Array angeordnet. Ihr Licht wird mittels einer Kollimatorlinse zu einem parallelen Lichtbündel geformt, das eine von dem behandelten Abgas durchströmte Messkammer durchstrahlt und anschließend auf einen Detektor fokussiert wird. Mittels eines Strahlteilers zwischen Kollimatorlinse und Messkammer wird ein Teil des Lichts auf einen Monitordetektor gelenkt. Die Leuchtdioden werden im Wechsel ein- und ausgeschaltet, um das in dem Abgas enthaltene Schwefeldioxid bei der Absorptionswellenlänge 280 nm und Stickstoffdioxid bei der Absorptionswellenlänge 400 nm zu detektieren. Das Detektorsignal wird mit dem Signal des Monitordetektors normalisiert, bevor es zur Ermittlung der Schwefeldioxid- und Stickstoffdioxid- bzw. Stickoxid-Konzentrationen in dem Abgas ausgewertet wird. Die Temperatur der Leuchtdioden wird mittels eines Peltierelements auf einen konstanten Wert geregelt.

Aus der CN 105424631 A ist ein Gasanalysator bekannt, bei dem zur Messung des Stickstoffmonoxid-Gehalts in Umgebungsluft eine Probe der Umgebungsluft durch eine erste Messkammer und anschließend nach Behandlung mit Ozon durch eine zweite Messkammer geleitet und beides Mal die Stickstoffdioxid-Konzentration in der jeweiligen Messkammer ermittelt wird. Dazu wird jede der beiden Messküvetten von dem Licht jeweils einer Leuchtdiode bei einer Zentralwellenlänge von 460 nm durchstrahlt und das aus der jeweiligen Messküvette austretende Licht detektiert. Das Ozon wird extern erzeugt und der Umgebungsluftprobe vor Eintritt in die zweite Messkammer zugemischt, wobei das Stickstoffmonoxid mit Hilfe des Ozons vollständig in Stickstoffdioxid umgesetzt wird. Da die Umgebungsluftprobe durch die Ozon-Zumischung verdünnt wird, ergibt sich der zu messende Stickstoffmonoxid-Gehalt aus der Differenz der in den beiden Messkammern ermittelten Stickstoffdioxid-Konzentrationen, wobei die in der zweiten Messkammer ermittelte Stickstoffdioxid-Konzentration mit einem die Volumenströme der Probe und des Ozons beinhaltenden Faktor multipliziert wird.

Aus der WO 2015/181879 A1 ist ein Gasanalysator zur Messung von Stickstoffdioxid, Stickstoffmonoxid und Schwefeldioxid in einem Abgas bekannt. Der Gasanalysator weist eine von dem Abgas durchströmte Messkammer auf, durch die über einen Strahlteiler das Licht von zwei Leuchtdioden auf einen Detektor geführt ist. Durch stille elektrische Entladung aus Umgebungsluft wird Ozon zur Behandlung des Abgases erzeugt und diesem beigemischt. Die Ozonerzeugung bzw. der Ozongenerator werden durch eine Auswerteeinrichtung des Gasanalysators ein- und ausgeschaltet. Bei eingeschaltetem Ozongenerator werden die Stickstoffdioxid-Konzentration des behandelten Abgases photometrisch bei einer Zentralwellenlänge zwischen 350 nm und 500 nm und die Ozonkonzentration bei einer Zentralwellenlänge zwischen 240 nm und 330 nm ermittelt. Bei ausgeschaltetem Ozongenerator wird die Stickstoffdioxid-Konzentration des unbehandelten Abgases ermittelt. Anhand der bei sowohl eingeschaltetem als auch ausgeschaltetem Ozongenerator ermittelten Konzentrationen und unter Verwendung eines entweder bekannten oder mittels eines Ozonmessers in dem Ozongenerator gemessenen Wertes für das erzeugte Ozon wird die Stickstoffmonoxid-Konzentration des Abgases rechnerisch ermittelt. Die Zentralwellenlänge zwischen 240 nm und 330 nm, insbesondere bei 280 nm, kann dazu verwendet werden, bei ausgeschaltetem Ozongenerator Schwefeldioxid-Konzentration in dem Abgas zu ermitteln.

Aus der DE 20 2017 001743 U1 ist ein Gasanalysator zur Messung von Stickstoffdioxid und Schwefeldioxid in einem Abgas bekannt. Das Licht von zwei Leuchtdioden mit einer Emissionswellenlängen von 285 nm im Absorptionsbereich von Schwefeldioxid und 400 nm im Absorptionsbereich von Stickstoffdioxid wird über einen Strahlteiler unmittelbar auf einen Referenzdetektor und durch eine von dem Abgas durchströmte Messkammer hindurch auf einen Messdetektor geleitet.

Aus der US 2013/045541 A1 die Messung von Konzentrationen eines Gases in einem Abgas vor und nach einer Oxidationseinrichtung, z. B. die Messung von Sauerstoff mittels Infrarot-Absorptionsspektroskopie, bekannt.

Aus der US 5 806 305 A ist eine Einrichtung zur katalytischen Abgasnachbehandlung bei Brennkraftmaschinen (Otto- oder Dieselmotoren) bekannt, bei der dem Abgas vor der Behandlung in dem Katalysator Ozon zugeführt wird. Das Ozon wird in einem Ozongenerator aus Frischluft mittels UV-Licht, beispielsweise einer Quecksilberdampflampe, bei einer Wellenlänge von 185 nm erzeugt.

Aus der EP 1 020 620 B1 ist es bekannt, zu demselben Zweck das Ozon aus Frischluft oder dem Abgas mittels UV-Licht, Mikrowelle oder Funkenentladung zu erzeugen.

Aus der JP 2012 026331 A ist es bekannt, das zu demselben Zweck aus Frischluft erzeugte Ozon vor und nach der Abgasbehandlung jeweils mittels eines Ozon-Halbleitersensors zu messen und in Abhängigkeit davon die Ozonerzeugung zu steuern.

Aus der EP 2 157 421 A1 ist es bekannt, zur Untersuchung des Schwefelgehalts von Kraftstoffen eine Probe des Kraftstoffs zu verbrennen und die Konzentration von Schwefeldioxid in dem Abgas durch ein Ultraviolett-Fluoreszenz-Messverfahren zu bestimmen. Um die Beeinträchtigung der Messung durch Stickstoffmonoxid zu vermeiden, wird das Abgas zuvor in einem Behälter dem Licht (185 nm) einer Niederdruck-Quecksilberentladungslampe ausgesetzt, um aus dem Restsauerstoffgehalt Ozon zu erzeugen und damit Stickstoffmonoxid in Stickstoffdioxid umzusetzen.

Der Erfindung liegt die Aufgabe zugrunde, eine zuverlässige kontinuierliche Messung von Stickoxiden und Schwefeldioxid in Abgasen mit geringem gerätetechnischem Aufwand zu ermöglichen.

Gemäß der Erfindung wird die Aufgabe durch den in Anspruch 1 definierten Gasanalysator gelöst, von dem vorteilhafte Weiterbildungen in den Unteransprüchen angegeben sind.

Für eine möglichst vollständige Umsetzung von Stickstoffmonoxid in Stickstoffdioxid in der Oxidationseinrichtung muss das Ozon mit einem gewissen Überschuss erzeugt werden. Bei der aus der erwähnten Publikation von Ryoichi Higashi et al. bekannten anschließenden thermischen Zersetzung von Distickstoffpentoxid kann neben Stickstoffdioxid auch wieder unerwünschtes Stickstoffmonoxid entstehen, weswegen die Aufheizung des Abgases kontrolliert erfolgen sollte. Dabei oder aufgrund von Störungen kann auch die thermische Zersetzung des überschüssigen Ozons unvollständig erfolgen. In dem zur Messung der Schwefeldioxid-Konzentration des Abgases verwendeten mittleren Ultraviolett-Bereich liegt jedoch eine starke Überlappung der Absorptionsbande von Schwefeldioxid und Ozon vor. Da jedoch gemäß der Erfindung die erste Messkammer von dem unbehandelten Abgas durchströmt wird, ist eine Störung der dort erfolgenden Messung der Schwefeldioxid-Konzentration durch Ozonanteile ausgeschlossen.

In der ersten Messkammer wird die Stickstoffdioxid-Konzentration des unbehandelten Abgases und in der zweiten Messkammer zusätzlich zu der ursprünglichen Stickstoffdioxid-Konzentration die Konzentration des in Stickstoffdioxid umgesetzten Stickstoffmonoxids gemessen. Der erfindungsgemäße Gasanalysator ermittelt in vorteilhafter Weise aus beiden sowohl die Stickoxid-Konzentration (als Summe der Stickstoffdioxid- und Stickstoffmonoxid-Konzentration) als auch die Stickstoffmonoxid-Konzentration und damit natürlich auch die Stickstoffdioxid-Konzentration des Abgases.

Eine Überwachung und ggf. Steuerung der möglichst vollständig zu erfolgenden Umsetzung des in dem Abgas enthaltenen Stickstoffmonoxids in mit dem Gasanalysator messbares Stickstoffdioxid findet dadurch statt, dass eine im Wellenlängenbereich von 760 nm bis 765 nm strahlende schmalbandige Lichtquelle vorgesehen ist, deren Licht von der Strahlteileranordnung durch beide Messkammern hindurch auf die beiden Detektoren geleitet wird und in weiteren Signalanteilen der Detektorsignale resultiert, und dass die Auswerteeinrichtung aus dem weiteren Signalanteil des ersten Detektorsignals die Sauerstoff-Konzentration des Abgases in der ersten Messkammer und aus dem weiteren Signalanteil des zweiten Detektorsignals die Sauerstoff-Konzentration des Abgases in der zweiten Messkammer ermittelt und anhand beider ermittelter Sauerstoff-Konzentrationen die Umsetzung des Stickstoffmonoxids in Stickstoffdioxid überwacht. In der ersten Messkammer wird die Sauerstoff-Konzentration des unbehandelten Abgases und in der zweiten Messkammer die Sauerstoff-Konzentration des Abgases nach Erzeugung des Ozons und damit erfolgender Umsetzung von Stickstoffmonoxid in Stickstoffdioxid gemessen. Die Differenz beider Messungen stellt den Anteil des bei der Umsetzung NO + O3 -> NO2 + O2 verbrauchten Sauerstoffs dar und ist somit ein Maß für den Grad der Umsetzung. Wenn das Ozon nicht ausschließlich aus dem Restsauerstoffgehalt des Abgases erzeugt wird, kann beispielsweise der zusätzliche zugeführte Sauerstoff (z. B. Luftsauerstoff) mit dem Abgas zunächst durch die erste Messkammer geführt und dort gemessen werden, bevor er in die Oxidationseinrichtung gelangt.

Bei der schmalbandigen Lichtquelle kann es sich z. B. um eine wellenlängenabstimmbare Laserdiode oder eine Leuchtdiode mit einem nachgeordneten Filter, z. B. Interferenzfilter, handeln. Die Wellenlänge des von der Laserdiode erzeugten Lichts kann in bekannter Weise über eine Absorptionslinie des Sauerstoffs abgestimmt werden, wobei das von den Detektoren detektierte Licht (hier die weiteren Signalanteile der Detektorsignale) wellenlängenabhängig, z. B. nach den Verfahren der direkten Absorptionsspektroskopie oder der Wellenlängenmodulationsspektroskopie, ausgewertet wird.

Zur Referenzierung (Normierung) der Messungen ist die Strahlteileranordnung vorzugsweise dazu ausgebildet, einen Teil des Lichts der beiden Leuchtdioden und, falls vorhanden, der schmalbandigen Lichtquelle auf einen Referenzdetektor abzuzweigen, der ein Referenzsignal mit aus dem Licht der Leuchtdioden und ggf. der schmalbandigen Lichtquelle resultierenden Referenz-Signalanteilen erzeugt. In der Auswerteeinrichtung findet dann die Referenzierung der Signalanteile der Detektorsignale mit den zugehörigen Referenz-Signalanteilen statt, beispielsweise durch Quotientenbildung. Um im Falle der Leuchtdioden die unterschiedlichen Signalanteile der Detektorsignale und Referenz-Signalanteile unterscheiden zu können, können die Leuchtdioden im Zeitmultiplex nacheinander angesteuert werden oder ihr Licht kann bei der Erzeugung unterschiedlich moduliert und bei der Detektion z. B. phasensensitiv demoduliert werden.

Die Ozonerzeugung in der Oxidationseinrichtung kann auf unterschiedliche Weise erfolgen. Die in der Publikation von Ryoichi Higashi et al. erwähnte Ozonerzeugung aus Luftsauerstoff mittels elektrischer Entladung (Koronaentladung) hat den Nachteil, dass sich Stickoxidverbindungen bilden, die für die Stickoxid-Messung des Abgases unerwünscht sind. Die Oxidationseinrichtung des erfindungsgemäßen Gasanalysators weist daher vorzugsweise einen Ozongenerator mit einer hochenergetische Strahlung mit einer Wellenlänge kleiner als 240 nm erzeugenden Ultraviolett-Lichtquelle, beispielsweise eine elektrodenlose Eximerlampe oder eine Niederdruck-Quecksilberentladungslampe, eine von dem Abgas durchströmte Reaktionskammer und eine dieser nachgeordnete Heizkammer auf. Bei magerer Verbrennung mit Luftüberschuss kann das Ozon vollständig aus dem Restsauerstoffgehalt des Abgases erzeugt werden. In diesem Fall ist der der Ozongenerator in der von dem Abgas durchströmten und nach außen geschlossenen Reaktionskammer angeordnet. Die Reaktionskammer kann z. B. in Form eines innenverspiegelten Rohres (z. B. Aluminium-Rohr) ausgebildet sein, in dem sich die beispielsweise zylinderförmige Ultraviolett-Lichtquelle erstreckt. Zur Erhöhung der Leistung bei der Umsetzung von Stickstoffmonoxid in Stickstoffdioxid können mehrerer solcher Rohre parallelgeschaltet sein. Wenn die Leistung der Ultraviolett-Lichtquelle mit der Zeit nachlässt, kann dem Abgas zusätzlich Luftsauerstoff zugeführt werden. Wie bereits erwähnt, kann der in dem unbehandelten Abgas enthaltene Sauerstoff (Restsauerstoff und ggf. zugeführter Sauerstoff) in der ersten Messkammer gemessen werden.

Zur weiteren Erläuterung der Erfindung wird im Folgenden auf die Figuren der Zeichnung Bezug genommen; im Einzelnen zeigen
- Figur 1: ein erstes Ausführungsbeispiel des erfindungsgemäßen Gasanalysators,
- Figur 2: ein Ausführungsbeispiel des Ozongenerators,
- Figur 3: beispielhaft die Absorptionsspektren von Stickstoffdioxid und Schwefeldioxid sowie die Emissionsspektren der Leuchtdioden des Gasanalysators und
- Figur 4: ein zweites Ausführungsbeispiel des erfindungsgemäßen Gasanalysators.

Gleiche und gleichwirkende Teile sind mit jeweils denselben Bezugszeichen versehen.

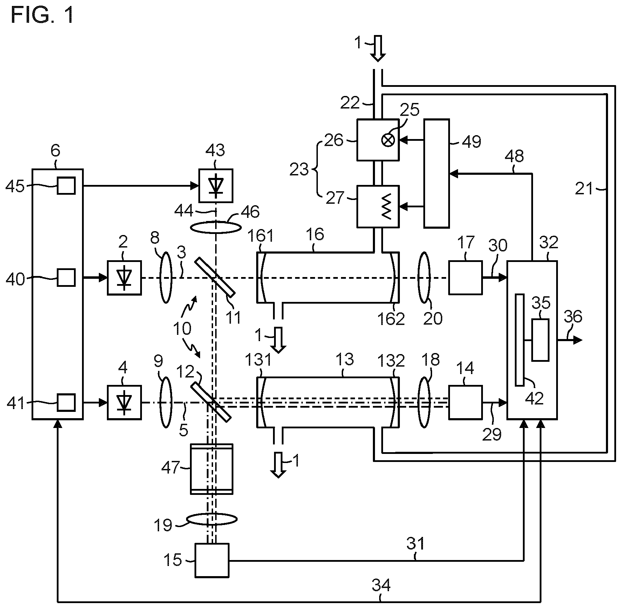

Figur 1 zeigt in vereinfachter schematischer Darstellung ein Blockschaltbild eines Gasanalysators zur Messung von Stickoxiden, Schwefeldioxid in einem Abgas 1. Eine erste Leuchtdiode 2 erzeugt Licht 3 im nahen Ultraviolett-Bereich zwischen 350 nm und 500 nm, beispielsweise um 405 nm. Eine zweite Leuchtdiode 4 erzeugt Licht 5 im mittleren Ultraviolett-Bereich zwischen 250 nm und 300 nm, beispielsweise 285 nm +/- 20 nm. Beide Leuchtdioden 2, 4 werden von einer Steuereinrichtung 6 angesteuert, die einen Multiplexer enthalten kann, um die Leuchtdioden 2, 4 abwechselnd ein- und auszuschalten, oder wie hier gezeigt Signalgeneratoren 40, 41 enthält, um die Leuchtdioden 2, 4 unterschiedlich zu modulieren, z. B. mit unterschiedlichen Modulationsfrequenzen, Taktraten oder Pulse-Codes. Das von den Leuchtdioden 2, 4 abgestrahlte Licht 3, 5 wird mit Hilfe von Kollimatorlinsen 8, 9 zu parallelen Lichtbündeln (im Weiteren als Lichtstrahlen bezeichnet) geformt und einer Strahlteileranordnung 10 mit zwei teildurchlässigen Spiegeln 11, 12, vorzugsweise sog. Polka-Dot-Strahlteiler mit konstantem Reflexions-/Transmissionsverhältnis über einen großen Spektralbereich, zugeführt. Der erste Spiegel 11 reflektiert einen Teil des Lichtstrahls 3 der ersten Leuchtdiode 2 zu dem zweiten Spiegel 12, der diesen Teil des Lichts 3 zusammen mit dem Licht 5 der zweiten Leuchtdiode 4 in einen Teilstrahl durch eine erste Messkammer 13 hindurch auf einen ersten Detektor 14 und einen weiteren Teilstrahl auf einen Referenzdetektor 15 aufteilt. Der von dem ersten Teilspiegel 11 durchgelassene Teil des Lichts 3 durchstrahlt eine zweite Messkammer 16 und fällt anschließend auf einen zweiten Detektor 17. Die jeweiligen Teilstrahlen werden mit Hilfe von Linsen 18, 19, 20 auf die Detektoren 14, 15, 17 fokussiert, bei denen es sich hier um Photodioden handelt.

Darüber hinaus ist im Bereich des teildurchlässigen Spiegels 11 der Strahlteileranordnung 10 eine schmalbandige Lichtquelle 43 angeordnet, die Licht 44 im Wellenlängenbereich von 760 nm bis 765 nm abstrahlt. Bei der Lichtquelle 43 kann es sich um eine Leuchtdiode oder wie hier im gezeigten Beispiel um eine Laserdiode handeln, die von einer Modulationseinrichtung 45 mit einer Stromrampe angesteuert wird, um mit dem erzeugten Licht 44 eine Sauerstoff-Absorptionslinie, beispielsweise bei 764 nm, wellenlängenabhängig abzutasten. Dabei kann der Strom zusätzlich sinusförmig moduliert werden. Das Licht 44 wird mittels einer Kollimatorlinse 46 zu einem parallelen Lichtbündel geformt, das von dem Spiegel 11 in einen Teilstrahl durch die zweite Messkammer 16 hindurch auf den zweiten Detektor 17 und einen weiteren Teilstrahl zu dem zweiten teildurchlässigen Spiegel 12 aufgeteilt wird. Dort findet eine weitere Aufteilung des Lichts 44 in einen Teilstrahl durch die erste Messkammer 13 hindurch auf den ersten Detektor 17 und einen weiteren Teilstrahl zu dem Referenzdetektor 15 statt. Eine optionale Referenzküvette 47 mit Sauerstoff-Füllung ermöglicht zudem eine zuverlässige Wellenlängenkalibrierung der Laserdiode 43.

Das Abgas 1 wird nach einer hier nicht gezeigten Vorbehandlung, die Entfeuchtung mittels Gaskühlung und Kondesatabscheidung sowie eine Partikelfeinfilterung umfasst, in zwei Teilströme aufgeteilt, von denen der eine Teilstrom über eine erste Gasleitung 21 direkt in die erste Messkammer 13 geleitet wird, während der andere Teilstrom über eine zweite Gasleitung 22 zunächst durch eine Oxidationseinrichtung 23 geleitet wird, bevor er in die zweite Messkammer 16 gelangt. Beide Abgas-Teilströme werden nach Durchströmen der jeweilige Messkammer 13 bzw. 16 aus dieser ausgeleitet. Der Gesamtstrom oder die beiden Teilströme können in an sich bekannter Weise durch Druck- oder Strömungsregelung auf vorgegebene Durchflusswerte eingestellt und konstant gehalten werden.

Die Oxidationseinrichtung 23 weist einen Ozongenerator 24 in Form einer Ultraviolett-Lichtquelle 25 in einer von dem Abgas 1 durchströmten geschlossenen Reaktionskammer 26 sowie eine sich anschließende Heizkammer 27 auf. Die Ultraviolett-Lichtquelle 25 erzeugt mit ihrer Strahlung von beispielsweise 185 nm aus dem Restsauerstoff des Abgases 1 Ozon, das in der Reaktionskammer 26 in dem Abgas 1 enthaltenes Stickstoffmonoxid in Stickstoffdioxid umsetzt. Jedoch reagiert auch Stickstoffdioxid mit dem erzeugten Ozon, so dass weitere Stickoxide, vor allem Distickstoffpentoxid entsteht. In der Heizkammer (Gasheizer) 27 werden diese unerwünschten Stickoxide sowie überschüssiges Ozon bei etwa 300 °C thermisch zu Stickstoffdioxid und Sauerstoff zersetzt.

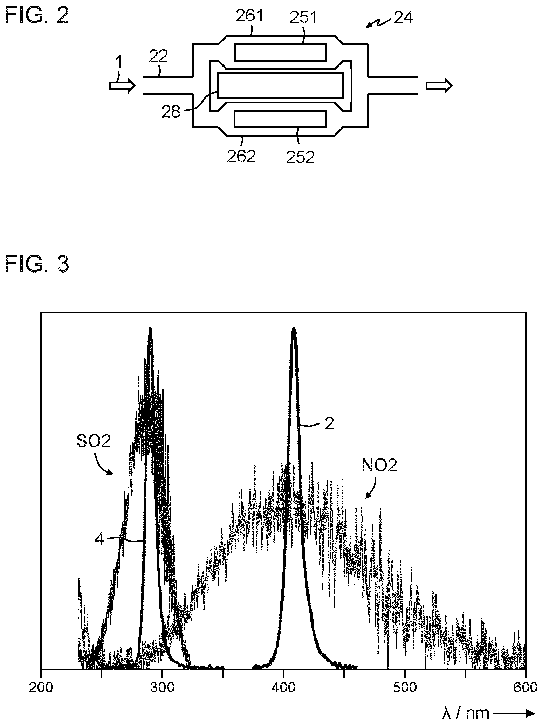

Figur 2 zeigt sehr vereinfacht ein Ausführungsbeispiel des Ozongenerators 24 mit hier zwei Ultraviolett-Lichtquellen 251, 252 in Form von zylinderförmigen Niederdruck-Quecksilberentladungslampen die in zwei parallel geschalteten und von dem Abgas 1 durchströmten innenverspiegelten Aluminium-Rohren 261, 261 angeordnet sind. Die Rohre 261, 261 bilden die Reaktionskammer 26. Die Anregung des Quecksilberplasmas kann über Elektroden oder, zur Erhöhung der Lebensdauer, elektrodenlos mittels eines Mikrowellengenerators 28 erfolgen.

Figur 3 zeigt beispielhaft die Absorptionsspektren von Stickstoffdioxid NO2 und Schwefeldioxid SO2, das Emissionsspektrum der ersten Leuchtdiode 2 bei 405 nm und das Emissionsspektrum der zweiten Leuchtdiode 4 bei 285 nm.

Zurück zu Figur 1 erzeugt der das Licht 3, 5 der Leuchtdioden 2, 4 nach Durchstrahlen der ersten Messkammer 13 mit dem unbehandelten Abgas 1 detektierende erste Detektor 14 ein erstes Detektorsignal 29 und der das Licht 3 der Leuchtdiode 2 nach Durchstrahlen der zweiten Messkammer 16 mit dem behandelten Abgas 1 detektierenden zweite Detektor 14 ein zweites Detektorsignal 30. Entsprechend der abwechselnden Ansteuerung der Leuchtdioden 2, 4 besteht das erstes Detektorsignal 29 aus zwei abwechselnd aufeinanderfolgenden Signalanteilen, von denen ein erster Signalanteil aus dem Licht 3 der ersten Leuchtdiode 2 und ein zweiter Signalanteil aus dem Licht der zweiten Leuchtdiode 4 resultiert. Das zweite Detektorsignal 30 weist nur einen aus dem Licht 3 der ersten Leuchtdiode 2 resultierenden Signalanteil auf. Der Referenzdetektor 15 erzeugt ein Referenzsignal 31 mit aus dem Licht 3, 5 beider Leuchtdioden 2, 4 resultierenden und abwechselnd aufeinanderfolgenden Referenz-Signalanteilen. Die Detektorsignale 29, 30 und das Referenzsignal 31 werden einer Auswerteeinrichtung 32 zugeführt, die einen Demodulator 42 enthält, der dazu ausgebildet ist, die Signalanteile der Detektorsignale 29, 30 und des Referenzsignals 31 bei den unterschiedlichen Modulationsfrequenzen oder Taktraten phasensensitiv zu demodulieren oder decodieren, um die Signalanteile für die weitere Verarbeitung und Auswertung zu trennen. Wenn die Leuchtdioden 2, 4 im Multiplexbetrieb angesteuert werden, enthält die Auswerteeinrichtung 32 dementsprechend anstelle des Demodulators 42 einen Demultiplexer. Die Synchronisierung des Multiplexers und Demultiplexers erfolgt dann über eine Kommunikationsleitung 34 zwischen der Steuereinrichtung 6 und der Auswerteeinrichtung 32. Nach Aufbereitung, z. B. Filterung und Digitalisierung, der Signale 29, 30, 31 werden in einer Recheneinrichtung 35 aus dem ersten Signalanteil des ersten Detektorsignals 29 die Stickstoffdioxid-Konzentration des unbehandelten Abgases in der ersten Messkammer 13 und aus dem zweiten Signalanteil die Schwefeldioxid-Konzentration ermittelt und als Ergebnis 36 der Gasanalysen ausgegeben. Aus dem zweiten Detektorsignal 30 wird die Stickstoffdioxid-Konzentration des behandelten Abgases in der zweiten Messkammer 16 ermittelt und in dem Ergebnis 36 als die Stickoxid-Konzentration des Abgases 1 ausgegeben. Dabei werden die Signalanteile der Detektorsignale 29, 30 mit den zugehörigen Signalanteilen des Referenzsignals 31 referenziert, so dass das Analysenergebnis 36 von der Helligkeit der Leuchtdioden 2, 3 und damit z. B. von ihrem Alterungszustand, unabhängig ist. Die Differenz der in den beidem Messkammern 13, 16 ermittelten Stickstoffdioxid-Konzentrationen entspricht der Stickstoffmonoxid-Konzentration des Abgases 1 und wird ebenfalls ausgegeben.

Trotz der erwähnten Referenzierung der Messungen ist es vorteilhaft, die gesamte photometrische Messanordnung des Gasanalysators zu thermostatieren. Dazu gehört auch eine Thermostatisierung der Leuchtdioden 2, 3 mit Hilfe von hier nicht gezeigten Peltierelementen, um Messbereiche im unteren ppm-Bereich realisieren zu können.

Der gezeigte Gasanalysator kann ohne Weiteres für die Messung weiterer Bestandteile des Abgases 1, wie z. B. Kohlendioxid, Kohlenmonoxid, Schwefel-, Chlor- und Iod-Verbindungen, erweitert werden. Anstelle der dazu erforderlichen weiteren geeigneten Lichtquellen (z. B. Leuchtdioden) können einzelne bereits vorhandene Leuchtdioden mit einem Leuchtstoff (Phosphor) versehen werden, der das von der betreffenden Leuchtdiode erzeugte Licht teilweise in ein Licht mit größerer Wellenlänge umwandelt. Dieses Prinzip ist beispielsweise aus der US 2010/049017 A1 bekannt.

Die beiden Leuchtdioden 2, 4 und ggf. weitere Leuchtdioden können in einem Array nebeneinander liegend angeordnet sein. Anstelle zweier Kollimatorlinsen für die verschiedenen Leuchtdioden 2, 4 ist dann nur eine gemeinsame Kollimatorlinse erforderlich.

Die unterschiedliche Anordnung der Leuchtdioden 2, 4 und ihre unterschiedliche Ansteuerung stellen voneinander unabhängige Maßnahmen dar, die dementsprechend einzeln oder zusammen angewandt werden können.

Die Detektion des Lichts 44 der Lichtquelle 43 durch die Detektoren 14, 17 führt zu weiteren Signalanteilen in den Detektorsignalen 29, 30, aus denen die Auswerteeinrichtung 32 die Sauerstoff-Konzentrationen des Abgases 1 in den beiden Messkammern 13, 16 ermittelt und im Rahmen des Analysenergebnisses 36 ausgibt. Dabei wird in der ersten Messkammer 13 die Sauerstoff-Konzentration des unbehandelten Abgases und in der zweiten Messkammer 16 die Sauerstoff-Konzentration des behandelten Abgases, also nach Erzeugung des Ozons und der damit erfolgenden Umsetzung von Stickstoffmonoxid in Stickstoffdioxid, gemessen. Die Differenz beider Sauerstoff-Messungen gibt daher den Anteil des bei der Umsetzung von Stickstoffmonoxid in Stickstoffdioxid verbrauchten Sauerstoffs dar und ist somit ein Maß für den Grad der Umsetzung. Diese Information kann als Steuersignal 48 für eine Steuereinrichtung 49 der Oxidationseinrichtung 23 verwendet werden. So kann z. B. die Leistung der Ultraviolett-Lichtquelle 25 oder die Heizleistung für die Gasaufheizung in der Heizkammer 27 optimiert oder geregelt werden. Aufgrund des niedrigen Absorptionsquerschnittes bei der Messung von Sauerstoff können die Messkammern 13, 16 als Multipass-Zellen ausgebildet werden, wobei anstelle von Fenstern hochreflektierende Konkavspiegel 131, 132, 161, 162 eingebaut sind, die für die Wellenlängen der Leuchtdioden 2 und 4 durchlässig sind.

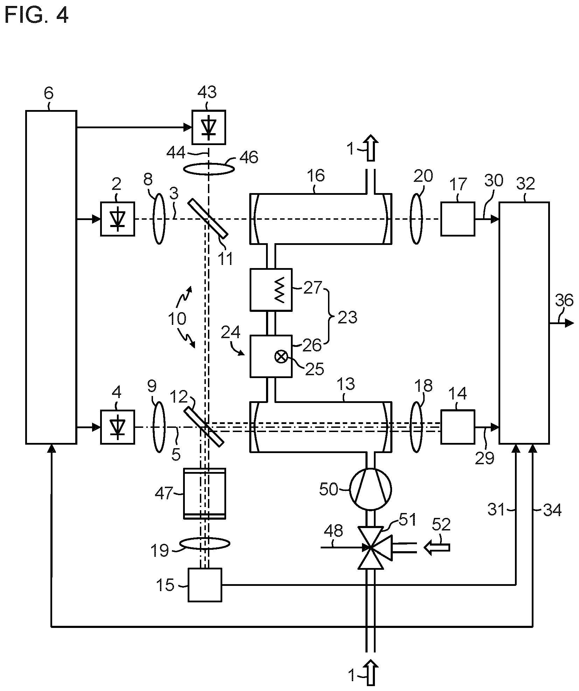

Figur 4 zeigt eine Abwandlung des Ausführungsbeispiels nach Figur 1, bei dem die erste Messkammer 13, die Oxidationseinrichtung 23 und die zweite Messkammer 16 hintereinander in Reihe geschaltet sind. Das Abgas 1 wird also nicht in zwei Teilströme durch die beiden Messkammern 13, 17 aufgeteilt, sondern zuerst unbehandelt durch die die erste Messkammer 13 geführt und danach behandelt, um schließlich die zweite Messkammer 17 zu durchströmen. Der Durchfluss des Abgases 1 durch den Gasanalysator wird mittels einer Gaspumpe 50 erzeugt und geregelt. Im Abgasweg vor der ersten Messkammer 13 ist ein steuerbares Dreiwege-Mischventil 51 angeordnet, um dem Abgas 1 Luftsauerstoff 52 beimischen zu können, wenn der Restsauerstoffgehalt des Abgases 1 oder die Leistung des Ozongenerators 24 für die zur vollständigen Umsetzung von Stickstoffmonoxid in Stickstoffdioxid erforderliche Ozonerzeugung nicht ausreicht. Die Beimischung kann manuell oder automatisch in Abhängigkeit von dem Steuersignal 48 (vgl. Figur 5) erfolgen.

## Patentansprüche

1. Gasanalysator zur Messung von Stickoxiden und Schwefeldioxid in einem Abgas (1) mit
- einer im nahen Ultraviolett-Bereich zwischen 350 nm und 500 nm strahlenden ersten Leuchtdiode (2),
- einer im mittleren Ultraviolett-Bereich zwischen 250 nm und 300 nm strahlenden zweiten Leuchtdiode (4),
- einer im Wellenlängenbereich von 760 nm bis 765 nm strahlenden schmalbandigen Lichtquelle (43)
- einer Oxidationseinrichtung (23), die zur Behandlung des Abgases (1) mit Ozon ausgebildet ist, um in dem Abgas (1) enthaltenes Stickstoffmonoxid in Stickstoffdioxid umzusetzen,
- einer von unbehandeltem Abgas (1) durchströmten und dem Licht (3, 5, 44) der beiden Leuchtdioden (2, 4) und der schmalbandigen Lichtquelle (43) durchstrahlten ersten Messkammer (13),
- einer an der Oxidationseinrichtung (23) angeschlossenen zweiten Messkammer (16), die von mit der Oxidationseinrichtung (23) behandeltem Abgas durchströmt wird,
- einem das Licht (3, 5, 44) der beiden Leuchtdioden (2, 4) und der schmalbandigen Lichtquelle (43) nach Durchstrahlen der ersten Messkammer (13) detektierenden ersten Detektor (14), welcher ein erstes Detektorsignal (29) mit einem aus dem Licht (3) der ersten Leuchtdiode (2) resultierenden ersten Signalanteil, einem aus dem Licht (5) der zweiten Leuchtdiode (4) resultierenden zweiten Signalanteil und einem aus dem Licht (44) der schmalbandigen Lichtquelle (43) resultierenden weiteren Signalanteil erzeugt,
- einer Strahlteileranordnung (10), die jeweils einen Teil des Lichts (3, 44) der ersten Leuchtdiode (2) und der schmalbandigen Lichtquelle (43) in die zweite Messkammer (16) abzweigt,
- einem das abgezweigte Licht (3, 44) der ersten Leuchtdiode (2) und der schmalbandigen Lichtquelle (43) nach Durchstrahlen der zweiten Messkammer (13) detektierenden zweiten Detektor (17), welcher ein zweites Detektorsignal (30) mit einem aus dem Licht (3) der ersten Leuchtdiode (2) resultierenden ersten Signalanteil und einem aus dem Licht (44) der schmalbandigen Lichtquelle (43) resultierenden weiteren Signalanteil erzeugt, und
- einer Auswerteeinrichtung (32) derart konfiguriert
- aus dem ersten Signalanteil des ersten Detektorsignals (29) die Stickstoffdioxid-Konzentration und aus dem zweiten Signalanteil des ersten Detektorsignals (29) die Schwefeldioxid-Konzentration des Abgases in der ersten Messkammer (13) zu ermitteln,
- ferner aus dem ersten Signalanteil des zweiten Detektorsignals (30) die Stickstoffdioxid-Konzentration des Abgases (1) in der zweiten Messkammer (16) zu ermitteln und als die Stickoxid-Konzentration des Abgases (1) auszugeben und die Differenz der in den beidem Messkammern (13, 16) ermittelten Stickstoffdioxid-Konzentrationen als Stickstoffmonoxid-Konzentration auszugeben, und
- aus dem weiteren Signalanteil des ersten Detektorsignals (29) die Sauerstoff-Konzentration des unbehandelten Abgases in der ersten Messkammer (13) und aus dem weiteren Signalanteil des zweiten Detektorsignals (30) die Sauerstoff-Konzentration des behandelten Abgases in der zweiten Messkammer (16) zu ermitteln und anhand beider ermittelter Sauerstoff-Konzentrationen die Umsetzung von Stickstoffmonoxid in Stickstoffdioxid in der Oxidationseinrichtung (23) zu überwachen.

2. Gasanalysator nach Anspruch 1, **dadurch gekennzeichnet, dass** die schmalbandige Lichtquelle (43) eine wellenlängenabstimmbare Laserdiode oder eine Leuchtdiode mit einem nachgeordneten Filter umfasst.

3. Gasanalysator nach Anspruch 1 oder 2, **dadurch gekennzeichnet, dass** die Messkammern (13, 16) als Multipass-Zellen mit hochreflektierenden und dabei für die Wellenlängen der ersten und zweiten Leuchtdiode (2, 4) durchlässigen Konkavspiegeln (131, 132, 161, 162) ausgebildet sind.

4. Gasanalysator nach einem der vorangehenden Ansprüche, **dadurch gekennzeichnet, dass** die Strahlteileranordnung (10) dazu ausgebildet ist, einen Teil des Lichts der beiden Leuchtdioden (2, 4) und der schmalbandigen Lichtquelle (43) auf einen Referenzdetektor (15) abzuzweigen, der ein Referenzsignal (31) mit aus dem Licht (3, 5) der Leuchtdioden (2, 4) und der schmalbandigen Lichtquelle (43) resultierenden Referenz-Signalanteilen erzeugt und dass die Auswerteeinrichtung (32) die Signalanteile der Detektorsignale (29, 30) mit den zugehörigen Referenz-Signalanteilen referenziert.

5. Gasanalysator nach einem der vorangehenden Ansprüche, **dadurch gekennzeichnet, dass** die Oxidationseinrichtung (23) einen Ozongenerator (24) mit einer Ultraviolett-Lichtquelle (25), eine von dem Abgas (1) durchströmte Reaktionskammer (26) und eine dieser nachgeordnete Heizkammer (27) aufweist.

6. Gasanalysator nach Anspruch 5, **dadurch gekennzeichnet, dass** die Ultraviolett-Lichtquelle (25; 251, 252) in der von dem Abgas (1) durchströmten geschlossenen Reaktionskammer (26; 261, 262) angeordnet ist und dazu ausgebildet ist, das Ozon aus dem Restsauerstoffgehalt des Abgases (1) zu erzeugen.

7. Gasanalysator nach einem der vorangehenden Ansprüche, **dadurch gekennzeichnet, dass** im Abgasweg vor der ersten Messkammer (13) und der Oxidationseinrichtung (23) ein Mischventil (51) angeordnet ist, das dazu ausgebildet ist, dem Abgas (1) Luftsauerstoff (52) beizumischen.

## Claims

1. Gas analyser for measuring nitrogen oxides and sulfur dioxide in an exhaust gas (1) with
- a first light-emitting diode (2) radiating in the near-ultraviolet range between 350 nm and 500 nm,
- a second light-emitting diode (4) radiating in the middle-ultraviolet range between 250 nm and 300 nm,
- a narrowband light source (43) which radiates in the wavelength range of 760 nm to 765 nm,
- an oxidation device (23), which is embodied for treating the exhaust gas (1) with ozone, in order to convert nitrogen monoxide contained in the exhaust gas (1) into nitrogen dioxide,
- a first measuring chamber (13), through which the untreated exhaust gas (1) flows and which is irradiated by the light (3, 5) of the two light-emitting diodes (2, 4) and the narrowband light source (43),
- a second measuring chamber (16) connected to the oxidation device (23), through which the exhaust gas treated by the oxidation device (23) flows,
- a first detector (14), which detects the light (3, 5, 44) of the two light-emitting diodes (2, 4) and the narrowband light source (43) after irradiation of the first measuring chamber (13) and which generates a first detector signal (29) with a first signal component resulting from the light (3) of the first light-emitting diode (2), a second signal component resulting from the light (5) of the second light-emitting diode (4) and a further signal component resulting from the light (44) of the narrowband light source (43),
- a beam splitter arrangement (10), which in each case splits off part of the light (3, 44) of the first light-emitting diode (2) and the narrowband light source (43) into the second measuring chamber (16),
- a second detector (17), which detects the split-off light (3, 44) of the first light-emitting diode (2) and the narrowband light source (43) after irradiation of the second measuring chamber (13) and which generates a second detector signal (30) with a first signal component resulting from the light (3) of the first light-emitting diode (2) and a further signal component resulting from the light (44) of the narrowband light source (43), and
- an evaluation device (32), which is configured to use the first signal component of the first detector signal (29) to ascertain the concentration of nitrogen dioxide and to use the second signal component of the first detector signal (29) to ascertain the concentration of sulfur dioxide in the exhaust gas in the first measuring chamber (13),
- furthermore to use the first signal component of the second detector signal (30) to ascertain the concentration of nitrogen dioxide in the exhaust gas (1) in the second measuring chamber (16) and to output this as the concentration of nitrogen oxide in the exhaust gas (1) and to output the difference in the concentration of nitrogen dioxide ascertained in the two measuring chambers (13, 16) as the concentration of nitrogen monoxide, and
- to use the further signal component of the first detector signal (29) to ascertain the concentration of oxygen in the untreated exhaust gas in the first measuring chamber (13) and to use the further signal component of the second detector signal (30) to ascertain the concentration of oxygen in the treated exhaust gas in the second measuring chamber (16), and, on the basis of both ascertained concentrations of oxygen, to monitor the conversion of the nitrogen monoxide into nitrogen dioxide in the oxidation device (23).

2. Gas analyser according to claim 1, **characterised in that** the narrowband light source (43) comprises a wavelength-tuneable laser diode or a light-emitting diode with a downstream filter.

3. Gas analyser according to claim 1 or 2, **characterised in that** the measuring chambers (13, 16) are embodied as multipass cells with highly reflective concave mirrors (131, 132, 161, 162) which are transparent for the wavelengths of the first and second light-emitting diode (2, 4).

4. Gas analyser according to one of the preceding claims, **characterised in that** the beam splitter arrangement (10) is embodied to split off part of the light of the two light-emitting diodes (2, 4) and the narrowband light source (43) to a reference detector (15), which generates a reference signal (31) using reference signal components resulting from the light (3, 5) of the light-emitting diodes (2, 4) and the narrowband light source (43) and that the evaluation device (32) references the signal components of the detector signals (29, 30) with the associated reference signal components.

5. Gas analyser according to one of the preceding claims, **characterised in that** the oxidation device (23) has an ozone generator (24) with an ultraviolet light source (25), a reaction chamber (26) through which the exhaust gas (1) flows and a heating chamber (27) downstream of said reaction chamber (26) .

6. Gas analyser according to claim 5, **characterised in that** the ultraviolet light source (25; 251, 252) is arranged in the closed reaction chamber (26; 261, 262) through which the exhaust gas (1) flows and is embodied to generate the ozone from the residual oxygen content of the exhaust gas (1).

7. Gas analyser according to one of the preceding claims, **characterised in that** a mixing valve (51), which is embodied to admix the exhaust gas (1) with atmospheric oxygen (52), is arranged in the exhaust gas path upstream of the first measuring chamber (13) and the oxidation device (23).

## Revendications

1. Analyseur de gaz dont la mesure d'oxydes d'azote et du dioxyde de soufre dans un gaz (1) d'échappement, comprenant
- une première diode (2) électroluminescente rayonnant dans le domaine de l'ultraviolet proche compris entre 350 nm et 500 nm,
- une deuxième diode (4) électroluminescente rayonnant dans le domaine de l'ultraviolet moyen compris entre 250 nm et 300 nm,
- une source (43) lumineuse à bande étroite rayonnant dans le domaine de longueur d'onde allant de 760 nm à 765 nm,
- un dispositif (23) d'oxydation, qui est constitué pour traiter le gaz (1) d'échappement par de l'ozone, afin de transformer en dioxyde d'azote le monoxyde d'azote contenu dans le gaz (1) d'échappement,
- une première chambre (13) de mesure, parcourue par du gaz (1) d'échappement non traité et traversée par la lumière (3, 5, 44) des deux diodes (2, 4) électroluminescentes et de la source (43)lumineuse à bande étroite,
- une deuxième chambre (16) de mesure, qui est raccordée à un dispositif (23) d'oxydation et qui est parcourue par du gaz d'échappement traité par le dispositif (23) d'oxydation,
- un premier détecteur (14), qui détecte la lumière (3, 5, 44) de deux diodes (2, 4) électroluminescentes et de la source (43) lumineuse à bande étroite après traversée de la première chambre (13) de mesure et qui produit un premier signal (29) de détecteur ayant une première proportion de signal provenant de la lumière (3) de la première diode (2) électroluminescente, une deuxième proportion de signal provenant de la lumière (5) de la deuxième diode (4) électroluminescente et une autre proportion de signal provenant de la lumière (44) de la source (43) lumineuse à bande étroite,
- un système (10) de diviseur de faisceau, qui fait bifurquer respectivement une partie de la lumière (3, 44) de la première diode (2) électroluminescente et de la source (43) lumineuse à bande étroite dans la deuxième chambre (16) de mesure,
- un deuxième détecteur (17), qui détecte la lumière (3, 44) ayant bifurqué de la première diode (2) électroluminescente et de la source (43) lumineuse à bande étroite après traversée de la deuxième chambre (13) de mesure et qui produit un deuxième signal (30) de détecteur ayant une première proportion de signal provenant de la lumière (30 de la première diode (2) électroluminescente et une autre proportion de signal provenant de la lumière (44) de la source (43) lumineuse à bande étroite,
et
- un dispositif (32) d'exploitation configuré de manière
- à déterminer, à partir de la première proportion de signal du premier signal (29) de détecteur, la concentration en dioxyde d'azote et, à partir de la deuxième proportion de signal du premier signal (29) de détecteur, la concentration en dioxyde de soufre du gaz d'échappement dans la première chambre (13) de mesure,
- à déterminer, en outre, à partir de la première proportion de signal du deuxième signal (30) de détecteur, la concentration en dioxyde d'azote du gaz (1) d'échappement dans la deuxième chambre (16) de mesure et
à la donner comme concentration d'oxyde d'azote du gaz (1) d'échappement
et à donner, comme concentration en monoxyde d'azote, la différence des concentrations de dioxyde d'azote déterminée dans les deux chambres (13, 16) de mesure,
et
- à déterminer, à partir de l'autre proportion de signal du premier signal (29) de détecteur, la concentration en oxygène du gaz d'échappement non traité dans la première chambre (13) de mesure et, à partir de l'autre proportion de signal du deuxième signal (32) détecteur, la concentration en oxygène du gaz d'échappement traité dans la deuxième chambre (16) de mesure
et, à l'aide des deux concentrations d'oxygène qui ont été déterminées, à contrôler la transformation du monoxyde d'azote en dioxyde d'azote dans le dispositif (23) d'oxydation.

2. Analyseur de gaz suivant la revendication 1, **caractérisé en ce que** la source (43) lumineuse à bande étroite comprend une diode laser pouvant être accordée en longueur d'onde ou une diode électroluminescente ayant un filtre en aval.

3. Analyseur de gaz suivant la revendication 1 ou 2, **caractérisé en ce que** les chambres (13, 16) de mesure sont constituées sous la forme de cellules multipasse ayant des miroirs (131, 132, 161, 162) concaves très réfléchissants et ainsi transparents aux longueurs d'onde de la première et de la deuxième diodes (2, 4) électroluminescentes.

4. Analyseur de gaz suivant l'une des revendications précédentes, **caractérisé en ce que** le système (10) de diviseur de faisceau est constitué pour faire bifurquer une partie de la lumière des diodes (2, 4) électroluminescentes et de la source (43) lumineuse à bande étroite sur un détecteur (15) de référence, qui produit un signal (31) de référence ayant des proportions de signal de référence provenant de la lumière (3, 5) des diodes de (4) électroluminescentes et de la source (43) lumineuse à bande étroite et **en ce que** le dispositif (32) d'exploitation référencie les proportions de signal des signaux (29, 30) de détecteur, par les proportions de signal de référence associées.

5. Analyseur de gaz suivant l'une des revendications précédentes, **caractérisé en ce que** le dispositif (23) d'oxydation a un générateur (24) d'ozone ayant une source (25) lumineuse ultraviolette, une chambre (26) de réaction parcourue par le gaz (1) d'échappement et une chambre (27) de chauffage en aval de celle-ci.

6. Analyseur de gaz suivant la revendication 5, **caractérisé en ce que** la source (25 ; 251, 252) lumineuse ultraviolette est disposée dans la chambre (26 ; 261, 262) de réaction fermée, parcourue par le gaz (1) d'échappement, et est constituée pour produire de l'ozone à partir de la teneur résiduelle en oxygène du gaz (1) d'échappement.

7. Analyseur de gaz suivant l'une des revendications précédentes, **caractérisé en ce que** dans le trajet du gaz d'échappement avant la première chambre (13) de mesure et le dispositif (23) d'oxydation est montée une vanne (51) de mélange, constituée pour mélanger de l'oxygène (52) de l'air au gaz (1) d'échappement.
